# EUROPEAN PATENT APPLICATION

(11) **EP 3 738 949 A1**
(43) Date of publication of application: **18.11.2020**
(21) Application number: 20177573.1
(22) Date of filing: 25.05.2016
(51) Int. Cl.: C07C 309/49, A61K 49/00, A61P 41/00, C09B 11/12, C09B 11/20

(54) **HEAVY DYE PREPARATIONS, IN PARTICULAR FOR USE IN SURGICAL METHODS OF OPHTHALMIC SURGERY AND FOR DYEING PROTEINS**

(30) Priority: 26.05.2015 IT UB20150864
(62) Divisional of application: 16734450.6
(71) Applicant: AL.CHI.MI.A. S.r.l., 35020 Ponte S. Nicolò (Padova) (IT)
(72) Inventor: Beccaro, Mauro, 35010 Cadoneghe (Padova) (IT); Bettini, Enrico, 30032 Fiesso D'Artico (Venezia) (IT); Signori, Paolo, 37100 Verona (IT)
(74) Representative: Ponchiroli, Simone

(57) **Abstract**

A dye preparation having a density greater than 1.01 g/cm³ and comprising at least one dye and at least one agent for increasing its density that is a polymer with the empirical formula (C₁₂H₂₂O₁₁·C₃H₅ClO)n, and the structure

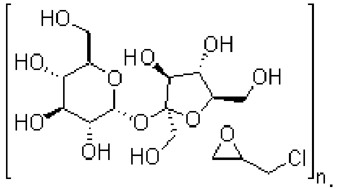

The dye is selected from the group consisting of triphenylmethane dyes, azo dyes, cyanine dyes, natural dyes and/or mixtures of them.

## Description

This invention relates to dye preparations comprising a new thickening agent, both as such and for use in methods for the treatment of the bodies of humans or animals. Moreover, this invention relates to dye preparations for dyeing the internal limiting membrane (hereinafter referred to as ILM) of an eye, as well as at least several types of epiretinal membranes (hereinafter referred to as ERPM), with the aim of creating a colour difference between the membranes dyed in that way (ILM and EPRM) and the underlying tissue during vitreoretinal surgeries involving the removal of such membranes. Furthermore, the dye preparation of this invention is intended to be used for dyeing protein chains. Finally, this invention relates to the use of dye preparations comprising the new thickening agent, both of the medical and non-medical type. In the context of this invention, also described is a new dye molecule, in particular a molecule usable for making preparations for dyeing the internal limiting membrane (hereinafter referred to as ILM) of an eye, as well as at least several types of epiretinal membranes (hereinafter referred to as ERPM), with the aim of creating a colour difference between the membranes dyed in that way (ILM and EPRM) and the underlying tissue during vitreoretinal surgeries involving the removal of such membranes. Furthermore, that new dye molecule is intended to be used for dyeing protein chains. This description also describes a dye preparation that comprises said new molecule, in particular for medical use, and even more particularly for the medical uses indicated above. Furthermore, there is a description of use of a preparation comprising said dye molecule, both in the medical sector, in particular for the specific medical uses indicated above, and in the non-medical sector for dyeing proteins.

As is fully described in the prior documents EP 1819366 A1 or US 2011/190728 A1, in many pathologies of the eye, vitrectomy surgery is often the best solution. However, during the surgery, it is important to minimise the risk of causing damage to the retina. One of the generally recognised precautionary measures consists of removing, during the surgery, the ILM, and if necessary any epiretinal membrane that has formed on it, in order to prevent intravitreal tensions from affecting the macula. According to the surgical technique generally used now, removal of the ILM and any epiretinal membranes is performed by mechanically detaching them using a suitable gripper, with a mechanical action called peeling. However, consequently, it has been realised how, for the surgeon, it is essential to be able to distinguish as much as possible between the membrane to be removed and the underlying retina.

For this reason, over the years, similarly to what occurs for other ophthalmic surgeries, it was suggested that the membranes to be removed should be selectively dyed, so that they can be visually distinguished from undyed underlying structures. Obviously, in order to be able to be used for that purpose, a dye must meet many requirements. In particular, on one hand it must be biocompatible and not cytotoxic or harmful to cells. On the other hand, it must preferably be soluble in water, it must be able to dye the membranes as selectively as possible and it must be easily flushed out of the eye at the end of the surgery, but not too easily during the surgery.

Many dyes have been proposed for this purpose. The initial dyes used included Indocyanine Green (hereinafter referred to as ICG - see for example "Indocyanine green-assisted peeling of the retinal internal limiting membrane". Burk SE et al. Ophthalmology. 2000;107:2010-2014) and Trypan Blue (hereinafter referred to as TB). However, the various studies carried out have highlighted several problems with such dyes. In particular, whilst for ICG many doubts were raised about its safety for use in human eyes, TB proved able to satisfactorily dye epiretinal membranes but not the ILM.

Other dyes were subsequently put forward, such as Brilliant Blue G (hereinafter referred to as BBG), Brilliant Blue R (hereinafter referred to as BBR), Patent Blue V or Methylene Blue.

In particular, in EP 1819366 A1 its was proposed that BBG, a salt of BBG or a hydrate of BBG should be used.

BBG is also indicated as the preferred dye in US 2011/190728 A1, a more generic document that describes the use of at least one dye selected from triphenylmethane dyes and/or azo dyes and/or cyanine dyes and/or natural dyes such as anthocyans and anthocyanidines.

However, of all of the various dyes indicated above, the only one that was subsequently marketed for dyeing the ILM, is BBG (which, it is no coincidence, is indicated as the preferred dye in the above-mentioned patent documents).

However, even that dye did not prove to be free of disadvantages.

In particular, BBG is a dye that is difficult to synthesize with high levels of purity, at least at a reasonable cost in commercial terms.

In fact, BBG is obtained by modification of BBR and the yields of the synthesis reaction are limited, therefore, the commercially available product very often has a level of purity that is even well below the declared 90% (tests conducted by the Applicant even showed levels of purity of 80%) with high contamination by the starting BBR. It is easy to imagine that, without a preliminary purity test, it can be difficult to make BBG-based dye preparations that have a precisely controlled dye content.

At the same time, attempting to further purify BBG is not advantageous in terms of the cost-benefit ratio.

In addition to these disadvantages, it should be noticed that BBG is not an intrinsically biocompatible substance, but is cyctotoxic. The possibility of using it or not in preparations intended for medical use or in any case for dyeing living material, depends exclusively on its concentration. Based on the scientific literature now available, in particular, BBG is considered not to be cytotoxic only when its concentration is not higher than 0.3 g/l (equal to 0.03% w/v - see tests carried out in accordance with DIN EN ISO 10993 shown on the sheet of the commercial product Brilliant Peel® from the company Geuder AG). However, according to many surgeons, at those concentrations the dyeing effect that can be achieved may not always be satisfactory, for example in the case of the eyes of people who are very short-sighted.

As already indicated, a second field of application of this invention is the generic dyeing of proteins and protein chains.

In fact, even in this sector, one of the most used dyes today is BBG.

However, the Applicant has realised that although it can dye proteins, BBG actually has a relatively low affinity with the self-same proteins. Tests performed on the protein commonly accepted as the reference protein (egg albumin), by means of circular dichroism (hereinafter CD) analysis, in fact highlighted for BBG-250 in a phosphate buffer (1.22 mg/ml in D₂O) an association constant Kₐ between BBG and albumin of approximately 38000 M⁻¹. It should be noticed that, to carry out the tests, the commercial BBG with a purity of 80% was preliminarily purified and the CD spectra were measured in the 800-400 nm region in the presence of increasing quantities of albumin. Finally, in the known way, the value of the association constant was determined using a non-linear regression of the intensity of the dichroic signal expressed in ΔA (A_{L}-A_{R}, where A_{L} and A_{R} respectively indicate the absorbency of the preparation as regards the two circularly polarized light waves used for the circular dichroism study, the left wave and the right wave) depending on the concentration of the egg white albumin, used as a reference. However, on this point what is indicated in the subsequent Affinity Tests section should also be considered.

In document US 2011/190728 A1 the dye preparation based on the selected dye is also made with a density of between 1.01 g/cm³ and 1.5 g/cm³, preferably between 1.01 g/cm³ and 1.3 g/cm³, in order to guarantee greater contact during the surgery between the dyed solution and the ILM and to overcome problems of excessive flushing away of the dye which may occur with dyes having a lower density, due to the continuous irrigation of the eye during the surgery.

According to that document, the agent used to increase the density is selected from heavy water D₂O, disaccharides or polysaccharides, or neutral polymers such as polyethers, polyvinyl alcohol, polyesters, polyacrylic acid copolymers and polyvinyl pyrrolidone.

In this context, the technical purpose which forms the basis of this invention is to provide a dye preparation with density greater than 1.01 g/cm³ which is alternative to those of the prior art.

Another technical purpose of this invention is to provide a heavy dye preparation that has better dyeing capability than the prior art ones.

Not least, the technical purpose of this invention is to provide a dye preparation that can be used in methods for treatment of the bodies of humans or animals.

The technical purpose specified and the aims indicated are substantially achieved by a dye molecule and by a dye preparation which comprises it, as described in the appended claims.

Further features and the advantages of this invention are more apparent in the detailed description with reference to several preferred, non-limiting embodiments of this invention illustrated later in this description.

Also provided in the context of this invention is a family of new dye molecules that is represented by the following structure (I): 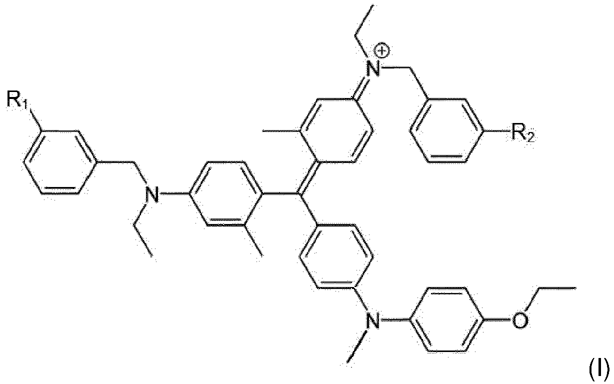 where R₂ is constituted of an SO₃⁻ group (which forms an inner salt with the nearby positively charged nitrogen N atom) and R₁ is constituted of an SO₃⁻ group bound with an ionic bond to a hydrogen H atom or to another atom (for example, sodium Na) or to an ammonium NH₄ group or to a lysine salt or to an arginine salt or to a different monovalent cation.

As can be seen, the new molecule differs from a molecule of BBG (it should be noticed that in the context of this invention, that name is also intended to indicate any product identified by synonyms, such as Coomassie® brilliant blue, Acid Blue 90, C. I. 42655 and Brilliant Blue G 250, all uniquely identified by CAS number: 6104-58-1) due to the presence of a methyl group rather than a hydrogen atom bound to the disubstituted amino nitrogen atom.

Synthesis of the new molecule provided in the context of this invention may be achieved by means of a methylation reaction to the disubstituted amino nitrogen of a starting product constituted of Brilliant Blue G250. In particular, the reaction is particularly advantageous starting with commercial BBG which, as said, usually has a level of purity less than or equal to 90% and is contaminated with BBR. In fact, at the end of the methylation reaction and of the subsequent purification, the contamination with BBR has been eliminated.

Generally speaking, the synthesis method involves making the starting product react in one or more successive steps with sodium hydroxide and methyl iodide, if necessary varying their proportions to one another each time.

### EXAMPLE

The following is a description of a preferred production example. Obviously, the quantities indicated could be varied, preserving the proportions between the various substances, it being understood that the process described leads to the production of approximately 1.6 g of molecule, and the production of significantly larger quantities could require some modifications to the process for its industrial optimisation.

### Materials and Methods

### Reagents

| Reagents | Manufacturer | Quantity for a batch of approx. 1.6 grams |
|---|---|---|
| Brilliant Blue G-pure (BBG) | (Sigma Aldrich, B0770) | 2g |
| RP grade methanol | (Sigma Aldrich) | 50 ml |
| Sodium hydroxide | (Sigma Aldrich) | 0.20 g |
| Methyl iodide | (Sigma Aldrich) | 2.20 ml |
| HPLC grade acetonitrile | (Sigma Aldrich) | 4 l |
| Deionised water | | 10 l |
| UV grade trifluoroacetic acid | (Sigma Aldrich) | 40 ml |
| HPLC grade methanol | (Sigma Aldrich) | 4 l |

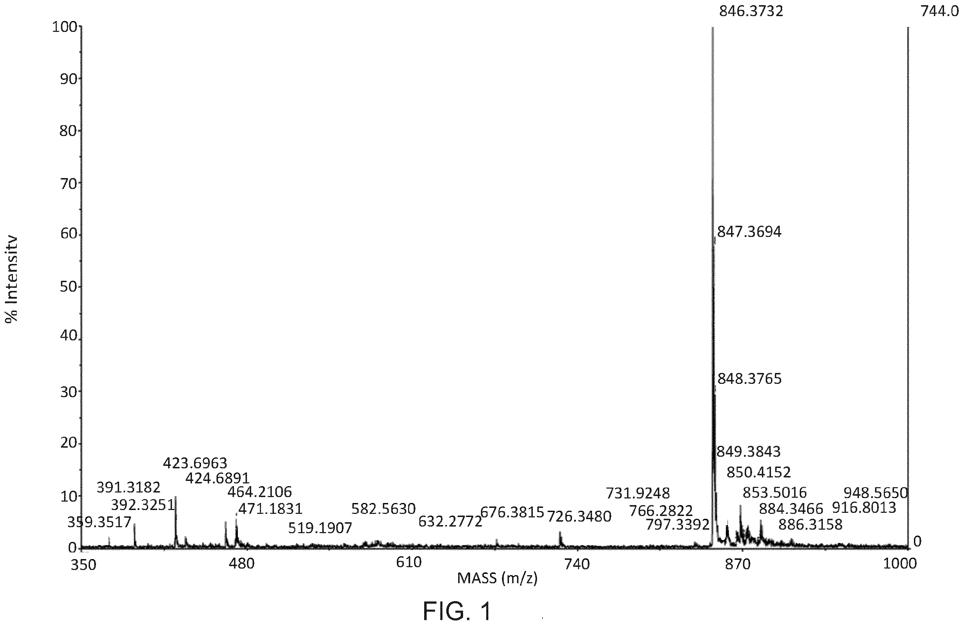

As regards the starting BBG, in the tests carried out it was a product with 91% purity (Analysis certificate B0770, Sigma Aldrich, batch SLBJ8621 V). Confirming that, Figure 1 shows its mass spectrum which indicates at least two significant peaks.

### Materials

| Materials | Quantity for a batch of approx. 1.6 grams |
|---|---|
| 250 ml glass flask | 1 |
| 5 ml pipettes | 2 |
| 500 ml, 1 I freeze-drying flasks | 5 |
| 16^{∗}160 mm glass test tubes washed in a laboratory washing machine | 300 |
| 200 ul vials for HPLC autosampler | 600 |

### Instruments

| Instruments | Manufacturer |
|---|---|
| Medium Pressure Liquid Chromatography | |
| REVELERIS C18 WP 40 g column | Grace |
| Freeze Dryer | Freeze Dryer Modulyo Edwards |
| HPLC | Shimadzu LC10 |
| Magnetic stirrer | Ika |
| Rotary evaporator | Buchi |

### Description of reaction

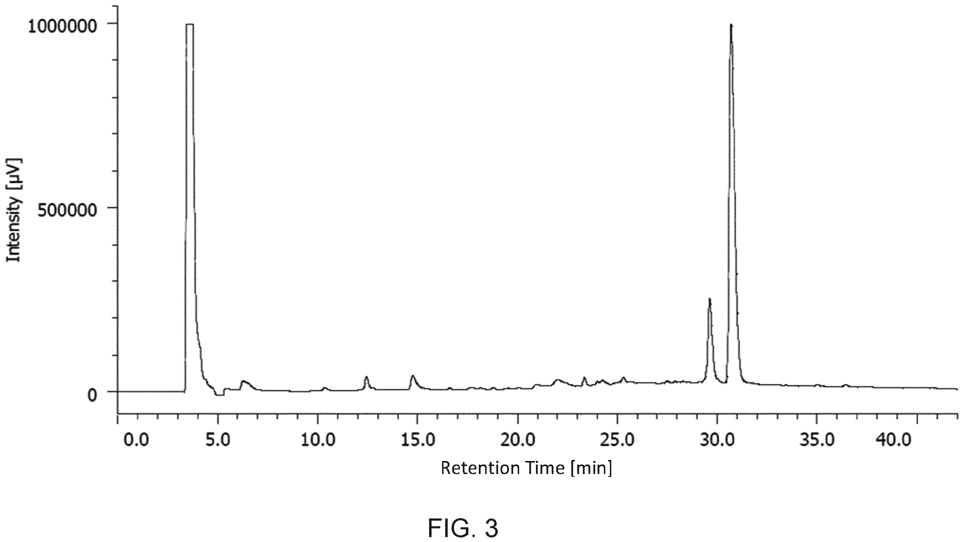

0.12 g of sodium hydroxide and 0.92 ml of methyl iodide are added to 1.7 g of BBG dissolved in methanol-water 1:1 v/v. The reaction is allowed to run for around two days at ambient temperature. The reaction mixture can then be checked using HPLC to highlight how the starting product is still present. The results of the HPLC analysis for the tests carried out are shown in Figure 3 and highlight three peaks that correspond, from left to right, the first to the solvent used in the reaction, the second to the molecule of BBG and the third to the new molecule developed in the context of this invention. At this point, another 0.92 ml of methyl iodide and 0.08 g of sodium hydroxide are added. After 7 days, another 0.36 ml of methyl iodide and 0.08 g of sodium hydroxide are added and the reaction is allowed to finish for another two days.
The reaction mixture is then concentrated in small volumes and the unrefined mixture is purified by means of Medium Pressure Liquid Chromatography (MPLC) using a REVELERIS C18 WP 40 g column (Grace) eluted with a mixture of water (A) and 90% v/v acetonitrile in water (B) both containing 0.05% v/v of trifluoroacetic acid.

After purification, the fractions containing the desired product are put together, concentrated in small volumes and freeze-dried. The solid dissolved in sodium hydroxide 0.01 is then purified using the same column with a mixture of water (A) - methanol (B). MPLC conditions: flow rate 20 ml/min, detection 254 nm, gradient 40% (B) for 5 min, 40-60% (B) in 40 min, 60-95% (B) in 2 min, 95% (B) 2 min, 95-40% (B) 2 min.

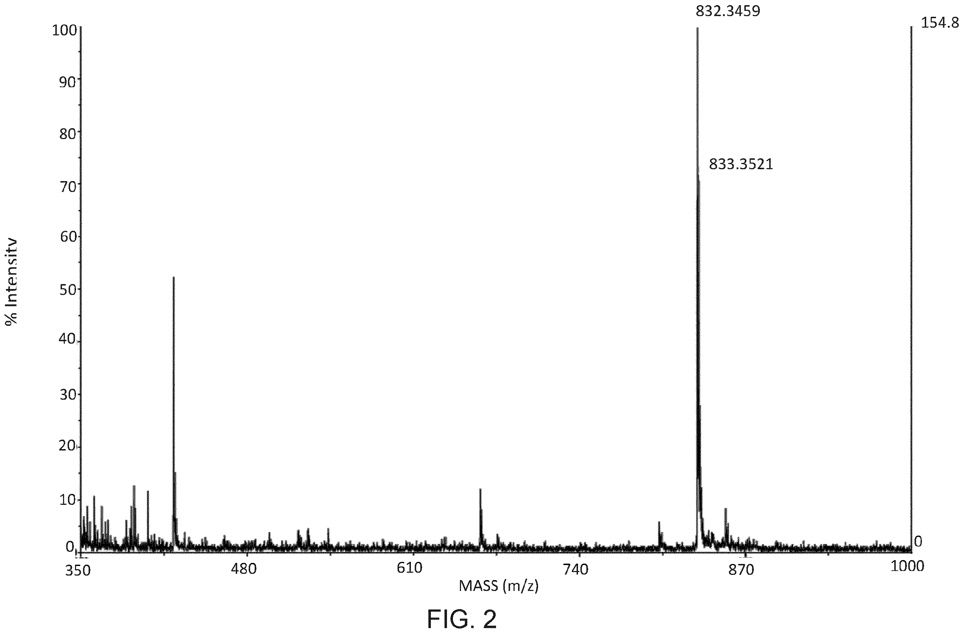

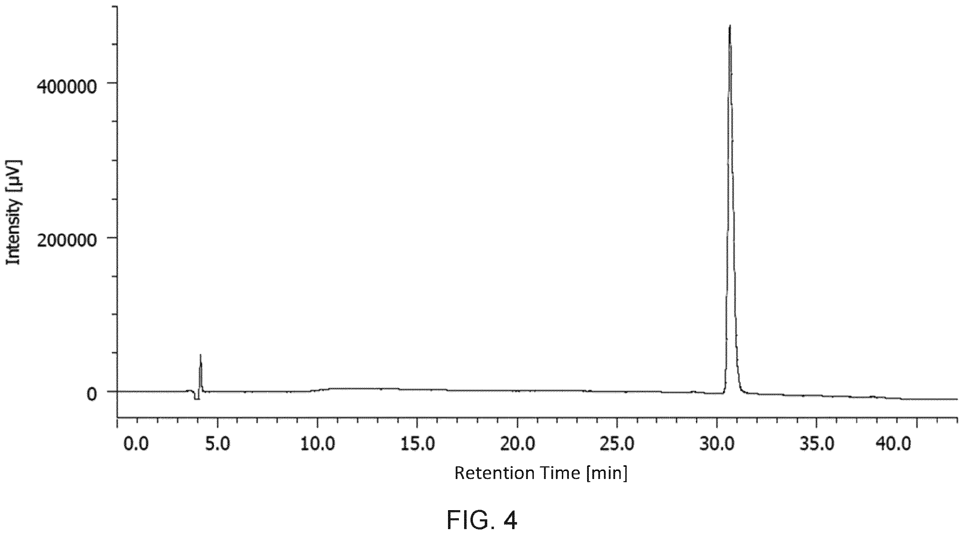

Figure 4 shows the result of the HPLC check on the purified and freeze-dried end product obtained in the tests carried out, and shows the presence of a single peak corresponding to the molecule developed in the context of this invention (in the form of sodium salt). Confirming that, Figure 2 shows the ESI-MS mass spectrum of the purified end product.
Also in the tests carried out, the purity of the end product was approximately 96.6% as calculated starting with the HPLC spectra, whilst the yield of the reaction was around 60% unrefined product (100 g of BBG-250 therefore yielded approximately 60 g of the new molecule, in the form of sodium salt).

Also developed in the context of this invention was a dye preparation comprising at least one first dye whose molecule is the new dye molecule, or a pharmaceutically acceptable salt of it or a hydrate of it.
Preferably, the dye preparation is an aqueous solution and/or has a phosphate buffered matrix. Moreover, it is liquid at least in the temperature range between 0°C and 50°C.
Moreover, at least for the uses in the context of methods for treatment of the bodies of humans or animals, the preparation is also advantageously sterile and biocompatible.
Advantageously, the first dye is present in a quantity, by weight relative to the total volume of the preparation (w/v), of between 0.0001% and 0.5%, preferably between 0.015% and 0.05%.
Moreover, in some preferred embodiments the dye preparation has a density of between 1.01 g/cm³ and 1.5 g/cm³ thanks to the additional presence of at least one agent for increasing its density.
The at least one agent for increasing the density may be selected according to requirements. For example, it may be selected in the group consisting of: heavy water D₂O, monosaccharides, disaccharides, polysaccharides, and neutral polymers; amongst the neutral polymers in particular it may be selected from polyethers, polyvinyl alcohol, polyesters, polyacrylic acid copolymers, mannitol and polyvinyl pyrrolidone.
However, in a particularly preferred embodiment the agent for increasing the density is a polymer with the empirical formula (C12H22O11•C3H5ClO)n, and structure 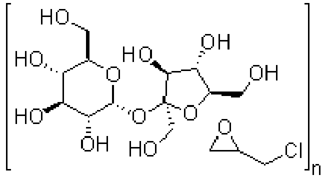 That agent is commercially known as Ficoll® or Polysucrose (CAS number: 26873-85-8), and is advantageously present in a quantity, by weight relative to the total volume of the preparation (w/v), of between 0.001% and 20%, preferably between 0.1% and 10%. Hereinafter, when reference is made to Ficoll, the intention is to indicate that agent.
In a particularly preferred formulation the preparation comprises 0.05% (w/v) of the molecule developed in the context of this invention and 4% (w/v) of Ficoll in a phosphate buffer.
Depending on the applications for which it is intended, the dye preparation may also comprise other substances, and in particular it may also comprise at least one second dye that is different to the first dye. In a preferred embodiment in which the dye preparation is intended to be used in the context of methods for treatment of the bodies of humans or animals, the second dye is advantageously trypan blue.
As regards the other properties of the dye preparation, such as dynamic viscosity, pH, osmolality, etc., in each case the expert in the field will be able to adapt them to the use of the dye preparation. For example, in the case of use of the dye preparation in the bodies of humans or animals, if necessary they can be adjusted in such a way that they are as close as possible to the physiological conditions.
As regards the possible uses of the dye preparation, one of those for which it is specifically intended is use in methods for treatment of the bodies of humans or animals.
Advantageously, in particular, the dye preparation is intended for use in a surgical method of vitreoretinal surgery.
Even more advantageously, the dye preparation is usable for dyeing the internal limiting membrane ILM and/or epiretinal membranes in a surgical method which involves subsequent removal of the ILM and/or of the EPRM.
In fact, it has been seen that the new dye molecule is able to selectively dye at least the ILM in such a way that during the step of detaching the ILM (peeling) and the EPRM, a clearly visible difference is created between the dyed ILM and the undyed underlying structures. To guarantee that all possible EPRM are also dyed, the dye preparation may also comprise a second dye, able to selectively dye them (such as trypan blue).
A further preferred use of the dye preparation is in a method in which it dyes proteins, for example to make a tissue to which the proteins belong more visible.

### EXPERIMENTAL DATA

The following are some experimental data obtained following tests and analyses carried out by the Applicant.

### Cytotoxicity analysis

The Applicant carried out various tests which highlighted how dye preparations developed in the context of this invention are not cytotoxic.

The following, by way of example, are the results obtained by the Eurofins Biolab S.r.l. laboratories in Vimodrone (Milan province), Italy, for three different batches of a dye preparation comprising 0.05% (w/v) of a molecule made in accordance with what is described above (with the group R₁ constituted of an SO₃⁻ group bound to a sodium cation), 4% (w/v) Ficoll in a phosphate buffer, which show how none of the three samples were cytotoxic.

It should be noticed how the concentration used is much higher than that (0.03 % w/v) at which BBG is declared to be cytotoxic.

| batch | sterilisation | % reduction in viability | | degree of cellular degeneration | |
|---|---|---|---|---|---|
| | | value measured | % cytotoxicity limit | value measured | degree cytotoxicity limit |
| 1 | autoclave 121.1 °C x20' | 1.6 | >30 | 0 | >2 |
| 2 | autoclave 121.1 °C x20' | -0.42 | >30 | 0 | >2 |
| 3 | autoclave 121.1 °C x20' | -2.77 | >30 | 0 | >2 |

### Affinity tests

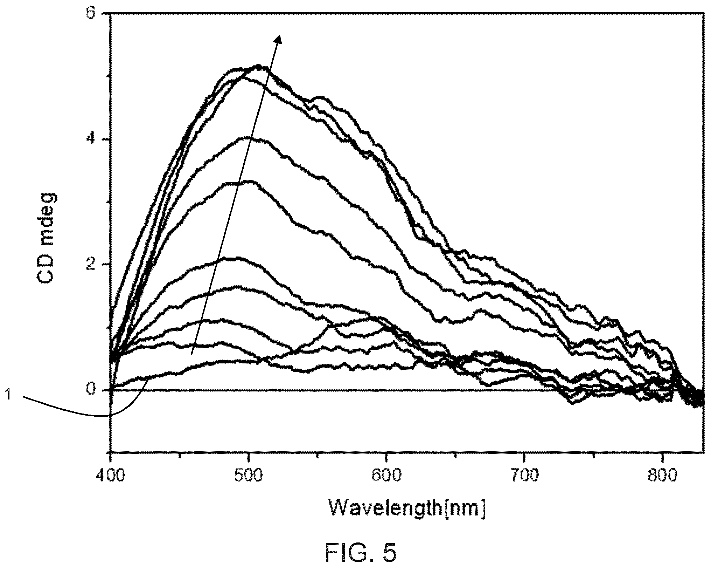

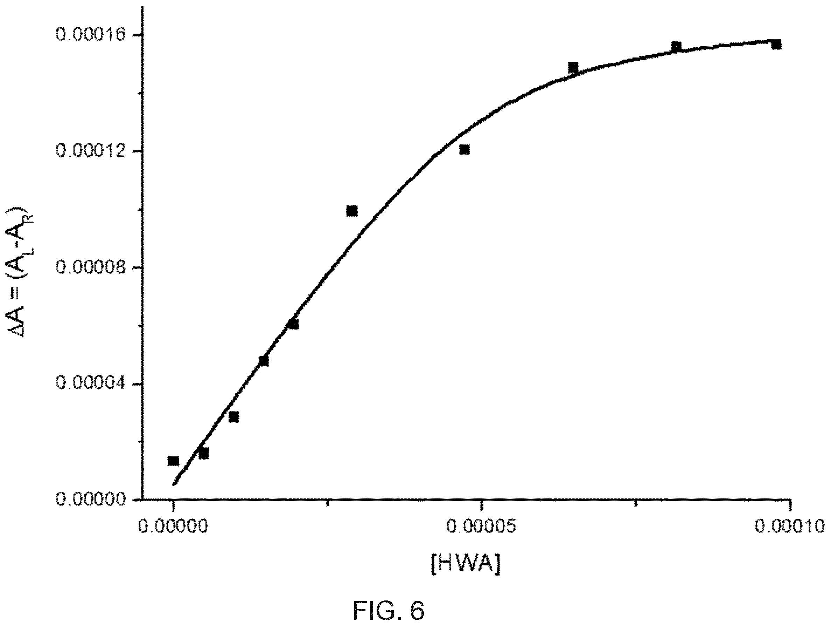

These are tests carried out by the Applicant to compare the new molecule developed in the context of this invention and commercial BBG-250, in terms of affinity with proteins and protein chains. As in the previous case, the molecule used in the tests is a molecule obtained with the method described in the Example, and in which the group R₁ is constituted of an SO₃⁻ group bound to a sodium cation.
In contrast, as regards the commercial BBG-250, given that the purity of said compound was approximately 91% (compared with the declared 80%), before carrying out the tests it was purified to approximately 98%.
In particular, tests were carried out on four different dye preparations, water-based in a phosphate buffer: for each molecule one comprising Ficoll (at 4% w/v) and one comprising heavy water D₂O (at 13% v/v). In all of the dye preparations tested, the phosphate buffer comprised:
- Sodium phosphate monobasic monohydrate NaH2P04 · H2O: 0.266 mg/ml;
- Sodium phosphate dibasic anhydrous Na2HPO4: 1.51 mg/ml;
- Sodium Chloride NaCl: 8.2 mg/ml.
Affinity was determined by means of CD (circular dichroism) spectroscopy. The CD spectra were measured in the 800-400 nm region in the presence of increasing quantities of egg white albumin which, as is known, for analyses of this type is commonly considered to be representative of proteins in general.
It should be noticed that in the conditions used (400-800 nm) the albumin does not absorb and therefore does not give rise to any dichroic signal, whilst the dyes have a high level of absorption, but do not show any dichroic signal. In contrast, the interaction of the albumin with the dye molecule (BBG-250 or the above-mentioned new molecule) induces a chirality in the dye that can be measured by means of CD. Consequently, titrating the dye with the albumin it is possible to determine its apparent binding constant or association constant, by means of a non-linear regression of the titration curve.
The CD spectroscopy data relative to a dye preparation comprising the new molecule and Ficoll are shown in Figure 5 where the number 1 indicates the dichroic spectrum of the dye preparation in the absence of protein, whilst the other lines relate to the same preparation in the presence of increasing quantities of albumin (according to the arrow).
As already indicated, using a non-linear regression of the intensity of the dichroic signal expressed in ΔA (A_{L}-A_{R}) depending on the concentration of egg white albumin, the values of the association constant Kₐ are determined. Figure 6 shows the trend of the regression concerning the dye preparation comprising the new molecule and Ficoll. It should be noticed that in the calculation of the association constant Kₐ the different concentrations of the two dyes was taken into account.
The results obtained are as follows:
- Dye preparation with BBG-250 in Ficoll 4% in a phosphate buffer (sterilised): Ka approximately 60,000 M⁻¹;
- Dye preparation with the new molecule in Ficoll 4% in a phosphate buffer (sterilised): Ka approximately 300,000 M⁻¹;
- Dye preparation with BBG-250 in a phosphate buffer (1.22 mg/ml in D₂0) (sterilised): Ka approximately 38,000 M⁻¹;
- Dye preparation with the new molecule in a phosphate buffer (0.58 mg/ml in D₂O) (sterilised): Ka approximately 180,000 M⁻¹.
Therefore, as can be seen, irrespective of the agent for increasing the density used, the new molecule developed in the context of this invention surprisingly showed an affinity for the albumin equal to 4.7-5 times that of commercial BBG-250.
Moreover, it was possible to establish that, for both of the dye molecules, the presence of Ficoll promoted (by 1.66 - 1.58 times) the association of the dye with the albumin compared with used in heavy water. Although the reason for this latter result is not currently known for sure, the theory of the Applicant is that the Ficoll, in addition to the role of agent for increasing the density, may also fulfil the role of "crowding agent" (hereinafter referred to as CA) and promote the interaction of the dyes with the egg albumin by means of "excluded volume" and spatial factors.
In fact, recently, Ficoll, together with PEG and dextran, has been studied and used as a CA, that is to say, as a filling/crowding agent, to mimic the conditions of the intracellular environment which is characterised by an extremely crowded environment, with a limited quantity of free water and an almost total absence of space. Many studies on CA (see, for example, "What Macromolecular Crowding Can Do to a Protein", Irina M. Kuznetsova 1,2, Konstantin K. Turoverov 1,2 and Vladimir N. Uversky 1,3,4,5) have in fact shown how "macromolecular crowding" created by the CA could affect the structure of the proteins, the folding, the form, shape, stability, the bond with the small molecules, enzymatic activity, protein-protein interactions, protein-nucleic acid interactions, and pathological aggregation.
The main mechanism of CA is to act for "excluded volume", which corresponds to spatial occupation and, therefore, to movement of the other molecules into the remaining free spaces. Locally this may result in an increase in concentration or in steric molecular changes of solutes. Therefore, this could also occur in the case in question.

In light of the experimental results just discussed concerning the surprising advantages than can be obtained using Ficoll as the agent for increasing the density, this invention relates to a dye preparation for use in a surgical method of ophthalmic surgery having a density higher than 1.01 g/cm³ and comprising Ficoll as an agent for increasing its density. That preparation may be used for all of the uses indicated above with reference to the new dye molecule described above.
Advantageously, this dye preparation may comprise at least one dye selected from the group consisting of: triphenylmethane dyes, azo dyes, cyanine dyes, natural dyes and/or mixtures of them, but preferably it will comprise BBG or a structural analogue of it.
Amongst the latter, those preferred are the pharmaceutically acceptable salts of BBG, BBG hydrates and BBG methylates.
From an operating viewpoint, in the case of use of a dyed preparation which uses Ficoll to obtain a density higher than 1.01 g/cm³, for dyeing the ILM, it should be remembered that in these surgeries following removal of the vitreous by means of the vitrectomy, the dye preparation is injected into the posterior chamber. Thanks to its high density it is deposited on the ILM with which it remains in contact only for several seconds before being removed by means of the usual abundant washing of the posterior chamber with BSS.

However, despite just several seconds of contact, the dye preparation dyes the ILM.
It should be noticed, however, that in these conditions of use (therefore even in the case of other ophthalmic surgeries performed under similar conditions), any Ficoll used as a thickener is not able to act as a CA, since the dye preparation is removed immediately after dyeing the membrane and is diluted in the posterior chamber with BSS.
Therefore, limited to the use for intraoperative dyeing in ophthalmic surgery methods, using Ficoll gives "only" performance similar to that obtainable, the density of the dye preparation being equal, with normal agents for increasing the density, such as heavy water.
This invention brings important advantages.
In fact, it has been possible to establish that the use of Ficoll as an agent for increasing the density, as an alternative to those used in the sector until now, in some applications allows results similar to those obtainable until now (as in the case of ophthalmic surgery), whilst in other applications it allows the use of less dye to achieve the same results.
Also regarding the new dye molecule provided in the context of this invention, first the great advantage was achieved of that molecule proving able to dye better than commercial BBG not just the ILM (circumstance declared by all of the surgeons involved in the experimental tests), but also the proteins and protein chains (see the results of the tests shown above), the consequence being that it is possible to use less dye to achieve the same results.
Moreover, it is not cytotoxic at concentrations significantly higher than those currently permitted for commercial BBG.
Finally, it should be noticed that this invention is relatively easy to produce and that even the cost linked to implementing the invention is not very high.
The invention described above may be modified and adapted in several ways without thereby departing from the scope of the inventive concept.

All details of the invention may be substituted with other technically equivalent elements and the materials used, as well as the shapes and dimensions of the various components, may vary according to requirements.

## Claims

1. A dye preparation having a density greater than 1.01 g/cm³ and comprising at least one dye and at least one agent for increasing its density that is a polymer with the empirical formula (C₁₂H₂₂O₁₁·C₃H₅ClO)x, and the structure

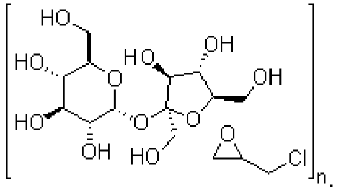

2. The dye preparation according to claim 1, comprising at least one first dye selected from the group consisting of azo dyes, cyanine dyes, natural dyes and/or mixtures of them.

3. The dye preparation according to claim 1, comprising at least one first dye selected from the group consisting of triphenylmethane dyes.

4. The dye preparation according to claim 3, wherein the first dye is Brilliant Blue G (BBG) or a structural analogue of it.

5. The dye preparation according to claim 3, wherein the first dye is selected from BBG, a pharmaceutically acceptable salt of BBG, a hydrate of BBG or a methylate of BBG.

6. The dye preparation according any one of claims 1 to 6, wherein the agent for increasing its density is present in a quantity, by weight relative to the total volume of the preparation (w/v), of between 0.001 % and 20%.

7. The dye preparation according to any one of claims 1 to 6, **characterised in that** it also comprises a phosphate buffered matrix.

8. The dye preparation according to any one of claims 1 to 7, **characterised in that** it is also liquid at least in the temperature range between 0°C and 50°C.

9. The dye preparation according to claim any one of claims 1 to 8, **characterised in that** the first dye is present in a quantity, by weight relative to the total volume of the preparation (w/v), of between 0.0001 % and 0.5%.

10. The dye preparation according to any one of claims 1 to 9, also comprising a second dye which is different to the first dye.

11. The dye preparation according to claim 10, wherein the second dye is trypan blue.

12. The dye preparation according to any one of claims 1 to 11 for use in methods for the treatment of the bodies of humans or animals.

13. The dye preparation according to claim 12, for use in a surgical method for vitreoretinal surgery.

14. The dye preparation according to claim 12 or 13, for use in dyeing the internal limiting membrane ILM and/or epiretinal membranes EPRM in a surgical method which involves subsequent removal respectively of the ILM and/or of the EPRM.

15. The dye preparation according to claim 14, when it is dependent on claim 10, for use also in dyeing any epiretinal membranes, wherein the first dye in use dyes at least the ILM and the second dye dyes the epiretinal membranes EPRM.

16. The dye preparation according to claim 12 for use in a method in which the dye preparation dyes proteins to make a tissue to which the proteins belong more visible.

17. Non-medical use of a dye preparation according to any one of claims 1 to 11 for dyeing proteins.
